# EUROPEAN PATENT APPLICATION

(11) **EP 2 910 193 A1**
(43) Date of publication of application: **26.08.2015**
(21) Application number: 14825078.0
(22) Date of filing: 12.11.2014
(51) Int. Cl.: A61B 10/00

(54) **BIOLOGICAL INFORMATION GENERATION METHOD, BIOLOGICAL INFORMATION GENERATION PROGRAM, AND BIOLOGICAL INFORMATION GENERATION DEVICE**

(30) Priority: 25.12.2013 JP 2013268117
(71) Applicant: Kusaba, Hiroki, Ehime 790-0062 (JP)
(72) Inventor: Kusaba, Hiroki, Ehime 790-0062 (JP)
(74) Representative: Hoffmann Eitle
(86) International application number: PCT/JP2014/079937
(87) International publication number: WO 2015/098331

(57) **Abstract**

Provided is a biological information generation method, a biological information generation program, and a biological information generation apparatus, which are capable of easily acquiring information on a biological condition by using a human's response to a smell.

A biological information generation method for generating graphic information for diagnosing a biological condition of a subject by using an olfactory stimulant includes: under a situation in which fragrant molecules are classified according to a height of a polarity and a charged state, a step of classifying the respective fragrant components capable of constituting olfactory stimulants into N types of classifications; a step of associating the fragrant components of each classification with an action thereof; a step of determining an unpleasure degree for olfactory stimulation given to the subject with respect to the N types of the olfactory stimulants; a step of assigning a correlation coefficient of the classification according to a main component in the N types of the olfactory stimulants and the actions associated with the corresponding classifications; and a step of generating graphic information for illustrating the biological condition of the subject with respect to each of the classifications.

## Description

### Technical Field

The present invention relates to a biological information generation method, a biological information generation program, and a biological information generation apparatus by olfactory stimulants.

### Background Art

In recent years, a correlation between a smell and a biological condition of a human (mental or physical condition) has drawn attention. For example, it is said that a smell of a lemon grass has a tranquilizing property, and there is high popularity on fields such as aroma therapy aiming to systematically understand efficacy of such smells and improve constitution by a smell of a plant.

Here, the correlation between such smells and the biological conditions of the human can also be used as a means different from the constitution improvement. For example, Patent Literature 1 discloses a technology that uses a smell as a tool for investigating the biological condition of the human. Specifically, Patent Literature 1 discloses a method that evaluates a human's subconscious response to a smell and a method that evaluates a subject's subconscious response to a fragrant stimulation.

### Citation List

### Patent Literature

Patent Literature 1: JP 2008-528122 T

### Summary of Invention

### Technical Problem

As such, the use of a data acquisition method according to Patent Literature 1 can investigate a human's subconscious mind based on a smell that is familiar information. However, since the data acquisition method according to Patent Literature 1 requires a step of evaluating a subject's bio field indicating a subject's individual energy field as a biophysical structure (for example, a step of capturing an image of a corona discharge from at least one fingertip of a subject by using a camera), a process becomes complicated and a dedicated measurement device is required.

Therefore, the present invention is directed to provide a biological information generation method, a biological information generation program, and a biological information generation apparatus, which are capable of easily acquiring information (biological information) on a biological condition by using a human's response to a smell.

### Solution to Problem

The present inventor has conducted intensive studies so as to solve the above-described problems. As a result, the present inventor completed the present invention by finding that information (in particular, graphic information) for understanding an overview of a biological condition of a subject can be generated by classifying fragrant molecules constituting olfactory stimulants according to polarity states thereof or charged states, associating the actions to the living body with respect to each classification, and appropriately digitizing a human's response to a smell based on the classifications and the actions. Thus, the present invention is as follows.

The present invention is a biological information generation method for generating graphic information for diagnosing a biological condition of a subject by using an olfactory stimulant, the biological information generation method including:
under a situation in which fragrant molecules are classified into first to M^{th} classifications (where M is a predetermined integer equal to or greater than 2) according to a height of a polarity and a charged state,
a first step of classifying the respective fragrant components capable of constituting olfactory stimulants into first to N classifications (where N is a predetermined integer equal to or greater than 2 and equal to or less than M) as a group including the fragrant molecules;
a second step of associating each of the first to N classifications with one or more actions that a fragrant component classified into each of the classifications gives to a biological condition;
a third step of determining an unpleasure degree for olfactory stimulation given to the subject for each of the N types of olfactory stimulants, with respect to at least N types of the olfactory stimulants including a first olfactory stimulant having the fragrant component classified into the first classification as a main component to an N olfactory stimulant having the fragrant component classified into the N classification as a main component;
a fourth step of assigning a relatively high value or a relatively low value to a correlation coefficient of the classification according to the main component in the corresponding type of the olfactory stimulant and the one or more actions associated with the corresponding classification with respect to the type of the olfactory stimulant, whose unpleasure degree is determined as being relatively high in the determination; and
a fifth step of generating graphic information for illustrating the biological condition of the subject with respect to each of the first to N classifications, based on the correlation coefficient.

Also, the present invention is a program for causing a computer to execute the biological information generation method, the program including:
a step of inputting an unpleasure degree determination result of olfactory stimulation given to the subject with respect to each of the N types of the olfactory stimulants;
a step of assigning a relatively high value or a relatively low value to the correlation coefficient of the classification according to the main component in the corresponding type of the olfactory stimulant and the one or more actions associated with the corresponding classification with respect to the type of the olfactory stimulant, whose unpleasure degree is determined as being relatively high in the input determination result; and
a step of generating the graphic information for illustrating the biological condition of the subject with respect to each of the first to N classifications, based on the correlation coefficient.

Furthermore, the present invention is an apparatus for executing the biological information generation method, the apparatus including:
an information input unit capable of inputting an unpleasure degree determination result of olfactory stimulation given to the subject with respect to each of the N types of the olfactory stimulants;
a correlation coefficient assignment unit for assigning a relatively high value or a relatively low value to the correlation coefficient of the classification according to the main component in the corresponding type of the olfactory stimulant and the one or more actions associated with the corresponding classification with respect to the type of the olfactory stimulant, whose unpleasure degree is determined as being relatively high in the input determination result; and
a graphic information generation unit for generating the graphic information for illustrating the biological condition of the subject with respect to each of the first to N classifications, based on the correlation coefficient.

### Advantageous Effects of Invention

According to the present invention, it is possible to provide a biological information generation method, a biological information generation program, and a biological information generation apparatus, which are capable of easily acquiring information (biological information) on a biological condition by using a human's response to a smell.

### Brief Description of Drawings

Fig. 1 is a conceptual diagram of a fragrant molecule classification method according to the present embodiment.

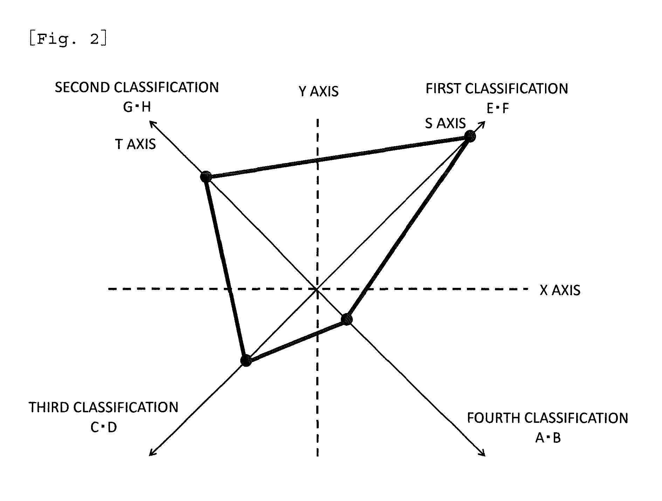

Fig. 2 is an example of graphic information for illustrating a biological condition according to the present embodiment.
Fig. 3 is a functional block diagram of a terminal T10 according to the present embodiment.
Fig. 4 is a flowchart of a program executed by the terminal T10 according to a first model of the present embodiment.
Fig. 5 is a flowchart of a recommended article derivation process according to the present embodiment.
Fig. 6 is a functional block diagram of a communication terminal T100 according to the present embodiment.
Fig. 7 is a functional block diagram of a web server V100 according to the present embodiment.
Fig. 8 is a system flow of the communication terminal T100 and the web server V100 according to the present embodiment.
Fig. 9 is an example of graphic information for illustrating a biological condition according to the present embodiment.
Fig. 10 is an example of graphic information for illustrating a biological condition according to the present embodiment.

### Description of Embodiments

The present invention includes a biological information generation information, a biological information generation program, and a biological information generation apparatus. Hereinafter, in the present invention, a principle related to an association between a smell and a biological condition will be described, and then, preferred embodiments of the biological information generation method, the biological information program, and the biological information generation apparatus will be described. Here, the following embodiments are only examples and the present invention is not limited to the following embodiments. Furthermore, information as specific examples described in the following embodiments are only examples and are not limited to the following examples. It should be understood that appropriate modifications can be made without departing from the intent of the following embodiments. Also, the configuration related to one item (for example, the biological information generation method) may be applied to another item (for example, the biological information generation program). Also, at least a part of the biological information generation method according to the present invention may be performed by a program, or may be performed by a circuit embedded in an apparatus.

### <<Principle related to association between smell and biological condition>>

First, in the present invention, the principle related to the association between the smell and the biological condition will be described.

A smell is felt when particular molecule stimulates an olfactory sense of a human (in the present embodiment, the molecule stimulating the olfactory sense of the human is referred to as a fragrant molecule, and in particular, a material containing the fragrant molecule is referred to as an olfactory stimulant). The olfactory sense is different from the other five senses in a way to be transmitted to a brain, and an olfactory cell is a sensory cell, is generated from the brain itself, and exists at a position closest to the outside world. Since smell information is processed only in a single layer in an olfactory bulb (a part of the brain), it is considered that the smell information is transmitted to a site responsible for emotion, without performing a smell recognition process. Thereby, a living body is instinctively driven to a pleasure-unpleasure emotion while not knowing what the smell is (that is, the smell is deeply connected to the pleasure-unpleasure emotion of the human). As such, regarding the association between the smell and the instinctive pleasure-unpleasure emotion of the human, it is known that a specific smell acts on the living body of the human and a certain efficacy is exhibited (for example, the smell of lemon grass promotes digestion).

Here, the present inventor focused attention on the fact that the human receives different impressions of smell according to the biological condition (for example, even in a case of the same smell, the human receives different impressions when feeling hungry and a full stomach), and inferred that when the human takes a certain smell and feels good, there is a growing desire for properties possessed by the smell (as a general example, in a case where it is determined that a smell having a fatigue recovery effect is a good smell, it is determined that fatigue is accumulated, or the like. That is, it is thought that the biological condition of the human can be known by investigating how the human responds when the human takes a smell combined with a specific property.

Since the property (action) of the smell is specified by the molecular characteristic of the fragrant molecule (for example, structural characteristic or property characteristic), the fragrant molecule can be previously classified into a plurality of types, based on the characteristic thereof, and the property of the smell can be associated with respect to each classification.

From the above, the present inventor assumed that it is possible to known the biological information of the human (what the living body of the human desires) by the molecular characteristic of the fragrant molecule and the response of the human to the corresponding fragrant molecule.

The present inventor recognized that, as a result of intensive research based on academic books or many statistics while mutually considering the action of the fragrant molecule and the molecular characteristic of the fragrant molecule, a two-axis classification method such as height of a polarity of a molecule and a charged state of a molecule (either positive or negative) can be exemplified as a method of classifying the molecular characteristics of the fragrant molecules, and the corresponding classification has a distinctive action on the living body with respect to each classification.

The charged state of the molecule mainly shows the charged state of the molecule, in particular, when the molecule is diffused (that is, at the time point when the olfactory sense of the human is stimulated), and is measured by the following method. That is, it includes a device that is connected to an ultrasonic head to generate ultrafine aerosol by sucking a fragrant liquid (essential oil, object, or aromatic water) in a capillary manner by a tassel of an absorbent cotton, or an ion supplement device (a box in which an opening of the supplement device is present toward the fragrant aerosol conically generated by the above device and an ion electrode is disposed inside the supplement device in a suspending manner). Also, the electrode is connected to a device (Kitore type current meter) that measures a slight current by a shielded cable. The device is installed to be grounded. When the device is in an operating state, the particle is discharged without interval with the formation of the aerosol, or it is gathered at an electrode form (through the ground). A current is generated and a difference of potential is accurately evaluated. Then, whether it is negative or positive is immediately recognized, and a value of charges brought by the particle is immediately measured.

In the classification method, various fragrant components are classified as illustrated in Table 1 below, according to the height of the polarity of the molecule and the charged state. Hereinafter, in the present embodiment, when the fragrant molecules are classified according to the height of the polarity of the molecule and the charged state, as illustrated in Table 1 below, a classification (first quadrant in Table 1) to which esters mainly belong is referred to as a first classification, a classification (second quadrant in Table 1) to which ketones mainly belong is referred to as a second classification, a classification (third quadrant in Table 1) to which phenols mainly belong is referred to as a third classification, and a classification (fourth quadrant in Table 1) to which hydrocarbons mainly belong is referred to as a fourth classification. In Table 1, an example of the fragrant molecules relevant to the classification method is illustrated, but other fragrant molecules can also be classified into any one of the classifications. Also, one molecule (for example, sesquiterpene hydrocarbons) exists over two quadrants (first quadrant and fourth quadrant) because, when seen as a more specific compound, its structural characteristic (the height of the polarity of the molecule and the charged state) and its property may be different. Table 2 below shows some commonly known efficacy of the fragrant molecules described in Table 1.

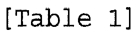

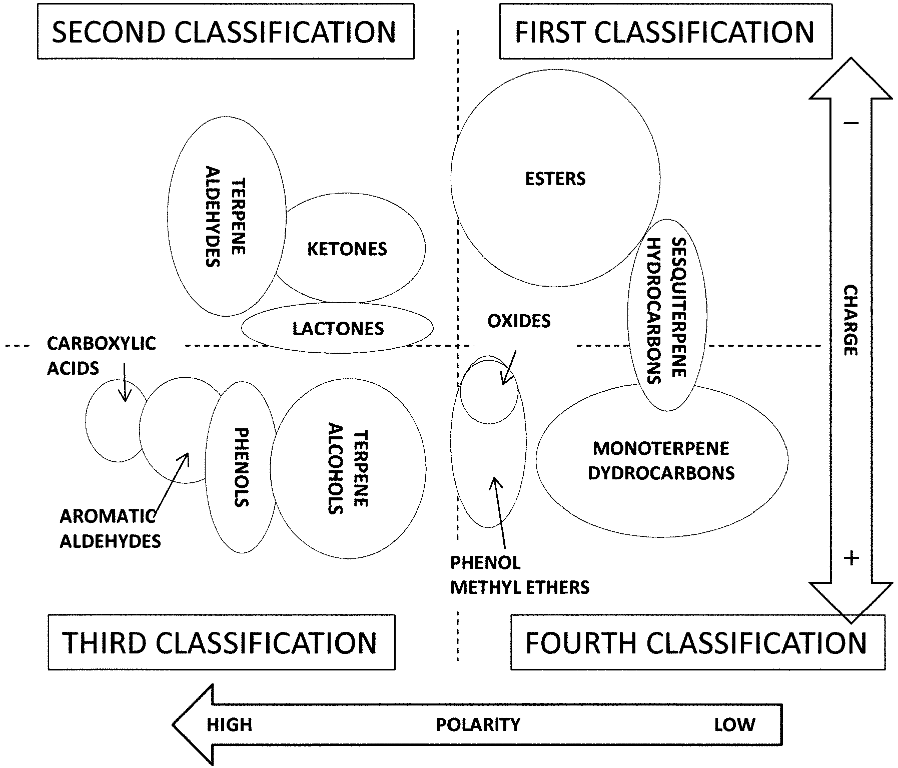

**[Table 2]**

| TERPENE ALDEHYDES | KETONES | ESTERS |
|---|---|---|
| ANTI-INFLAMMATORY ACTION | FAT DISSOLUTION ACTION | NERVE BALANCE RECOVERY ACTION |
| ANALGESIC ACTION | CHOLERETIC ACTION | BLOOD PRESSURE-LOWERING ACTIO |
| STONE DISSOLUTION ACTION | MUCOLYTIC ACTION | SEDATION ACTION |
| SEDATION ACTION | EXPECTORANT ACTION | ANTI-CONVULSION ACTION |
| DIGESTION PROMOTING ACTION | PHYTOPHTHORA MARK FORMIGN ACTION | ANALGESIC ACTION |
| BLOOD PRESSURE-LOWERING ACTION | | ANTI-INFLAMMATORY ACTION |
| ANTIFUNGAL ACTION | LACTONES | |
| ANTIVIRAL ACTION | FAT DISSOLUTION ACTION | SESQUITERPENE HYDROCARBONS (- |
| ANTIBACTERIAL ACTION | PHYTOPHTHORA MARK FORMIGN ACTION | SEDATION ACTION |
| | MUCOLYTIC ACTION ANTIVIRAL ACTION | ANTI-INFLAMMATORY ACTION |

| AROMATIC ALDEHYDES | TERPENE ALCOHOLS | SESQUITERPENE HYDROCARBONS(+) |
|---|---|---|
| ANTIBACTERIAL ACTION | STIMULATING ACTION | TONIC STIMULATING ACTION |
| ANTIVIRAL ACTION | ANTIBACTERIAL ACTION | |
| ANTIMYCOTIC ACTION | ANTIVIRAL ACTION | MONOTERPENE HYDROCARBONS |
| ANTIPARASITIC ACTION | ANTIMYCOTIC ACTION | STASIS REMOVAL ACTION |
| IMMUNOSTIMULATORY ACTION | TONIC ACTION | CORTISONE-LIKE ACTION |
| NERVE TONIC ACTION | HORMONE-LIKE ACTION | ANTI-INFLAMMATORY ACTION |
| FERMENTATION INHIBITORY ACTION | DECONGESTANT ACTION | ANTIVIRAL ACTION |
| | IMMUNOMODULATORY ACTION | ANTIBACTERIAL ACTION |
| PHENOLS | NERVE TONIC ACTION | |
| ANTIPARASITIC ACTION | ANTIPARASITIC ACTION | OXIDIZED SUBSTANCE |
| ANTIBACTERIAL ACTION | TONIC STIMULATING ACTION | SPUTUM REMOVAL ACTION |
| ANTIVIRAL ACTION | | ANTI-CATARRHAL ACTION |
| ANTIMYCOTIC ACTION | | ANTIVIRAL ACTION |
| TONIC ACTION | | IMMUNOMODULATORY ACTION |
| STIMULATING ACTION | | ANTIBACTERIAL ACTION |
| IMMUNOSTIMULATORY ACTION | | ANTIPARASITIC ACTION |
| WARMING ACTION | | |
| | | PHENOL METHYL ETHERS |
| | | ANTI-CONVULSION ACTION |
| | | ANALGESIC ACTION |
| | | ANTI-INFLAMMATORY ACTION |
| | | ANTIMYCOTIC ACTION |
| | | ANTIVIRAL ACTION |
| | | ANTIBACTERIAL ACTION |

This principle is based on the recognition that the response of the human to such fragrant molecules, in particular, the information of the pleasure-unpleasure (likes and dislikes) with respect to the smell thereof, corresponds to the biological condition of the human {that is, it is possible to grasp the biological condition of the human by finding how pleasant or unpleasant the olfactory stimulant whose fragrant component (fragrant molecule) is known is}. Based on the corresponding recognition, the following properties was confirmed by investigating the response and the biological condition of the human in relation to the fragrant molecules according to the first classification, the fragrant molecules according to the second classification, the fragrant molecules according to the third classification, and the fragrant molecules according to the fourth classification by using Table 1, Table 2, and statistical data.

In a case where the fragrant components according to the first classification (fragrant components to which esters belong) are felt as good smell, it is considered that the nerve balance recovery action being the component characteristic thereof is required, and it is easy to break the balance of the nerve and thus there is a disease or restless condition of the nervous system. Conversely, in a case where the fragrant components according to the first classification are felt as unpleasant smell, it may be considered that the nerve balance recovery is not required, and it can be regarded as a calm condition and it can be considered that there is a tendency to worry too much.

In a case where the fragrant components according to the second classification (fragrant components to which terpene aldehydes belong) are felt as good smell, it is considered that the digestion promoting action being the component characteristic thereof is required, and the digestive system is bad, the recovery of an injury or a cold is slow, and it is easy to suffer from stiff neck from the disorder of the stomach or the like. Conversely, in a case where the fragrant components according to the second classification are felt as unpleasant smell, it is considered that the digestion promoting action is not required, and there is a probability of overeating or excess of water important to promote digestion, it may be though that it is easily swollen and easily cold, and it may be thought that a behavior is slow or a waist is heavy.

In a case where the fragrant components according to the third classification (fragrant components to which terpene alcohols belong) are felt as good smell, it may be considered that the nerve tonic action being the component characteristic thereof is required, and the mood is hardly uplifted and the motivation does not come out. Conversely, in a case where the fragrant components according to the third classification are felt as unpleasant smell, it may be considered that the nerve tonic action is not required, and thus, care is growing, there is a motivation, and the nerve is in an unstable condition.

In a case where the fragrant components according to the fourth classification (fragrant components to which monoterpene hydrocarbons belong) are felt as good smell, it may be considered that the stasis removal action being the component characteristic thereof is required, and a function of discharging excess water from the body is weak, and symptoms such as swelling, chronic fatigue, cold, fatigue occur. Conversely, in a case where the fragrant components according to the fourth classification are felt as unpleasant smell, it may be considered that the stasis removal action is not required, and thus, metabolism is high and it is vigorous and active.

Here, an amount of fragrant molecules included in a certain olfactory stimulant can be quantitatively measured. That is, it is possible to quantitatively measure how much a certain olfactory stimulant contains fragrant molecules classified into the first classification, the second classification, the third classification, and the fourth classification.

In the present principle, which classification of the fragrant component is a main component of the fragrant component among the olfactory stimulants may be determined by measuring an amount of fragrant molecules belonging to each classification among the corresponding olfactory stimulants and comparing the amount of the fragrant molecules with respect to each classification. The fragrant components show a group including fragrant molecules, and the classified fragrant components (fragrant components according to a certain classification) show a group including fragrant molecules belonging to a certain classification (in the present principle, the first classification, the second classification, the third classification, and the fourth classification). For example, in a case where the fragrant molecules constituting the olfactory stimulants are phenols and carboxylic acids, the group including the fragrant molecules classified into the third classification among the olfactory stimulants are the summary of all the fragrant molecules (for example, phenols and carboxylic acids) belonging to the third classification (therefore, for example, the amount of the components according to the third classification is the sum of the amount of the phenols and the amount of the carboxylic acids). Also, that the classified fragrant components are the main component of the olfactory stimulant indicates that the corresponding fragrant components are present in an amount capable of constituting the smell of the olfactory stimulant (that is, an amount that can be consciously or unconsciously discriminated when the human takes a smell of the olfactory stimulant) {however, it may be determined by only a substantial amount of components classified into a certain classification (that is, a substantial total amount of the fragrant molecules classified into the corresponding classification (for example, there may be an indication that 50 wt% or more is contained in the total fragrant molecules}. Therefore, in the olfactory stimulants, a plurality of classified fragrant components may be the main component at the same time. In this case, the olfactory stimulant may have the plurality of classified fragrant components as the main component, or may have only the classified fragrant components having highest effect on the smell (or highest content) as the main component among the classified fragrant components constituting the corresponding olfactory stimulant (that is, it may be considered to have only the fragrant components classified into any one of the first classification, the second classification, the third classification, and the fourth classification). For example, regarding the fragrant components constituting a certain olfactory stimulant, in a case where the fragrant component according to the first classification occupies 50% of the total fragrant molecules and the fragrant component according to the third classification also occupies 50% of the total fragrant molecules in a mass conversion, only the fragrant component according to the third classification (or only the fragrant component according to the third classification) may be referenced, and the fragrant component according to the first classification and the fragrant component according to the third classification may be weighted by the influence on the smell (or, weighting may be simply performed by a mass ratio). Also, in a case where the main component of the fragrant component constituting the olfactory stimulant is only the fragrant component according to a certain classification (or when assumed as such), it is advantageous in that it becomes easy to perform data processing in a graphic information generating method or the like, or a diagnosis.

In the present principle, it is preferable that essential oil including natural ingredients is used as the olfactory stimulant. In a case of the essential oil including the natural ingredients, since the content ratio of the fragrant molecules has been analyzed, it is easy to infer a biological condition. On the other hand, in a case of artificial flavor, that is, essential oil to which component addition or removal is performed, the olfactory sense may cause an adverse reaction with respect to the artifact, and a biological condition may not be accurately inferred. As examples of the essential oil including the natural ingredients used in the present principle, there are known natural ingredients, such as Akamatsu Europe (Pinus sylvestris), palmarosa (Cymbopogon martini), lemon grass (Cymbopogon citratus), sage (Salvia officinalis), Chamomile Roman (Chamaemelum nobile), peppermint (Mentha x piperita), lavender spica (Lavandula spica), rosemary (Rosmarinus officinalis), wintergreen (Gaultheria procumbens), petitgrain (Citrus aurantium, eucalyptus lemon (Eucalyptus citriodora), lavender angustifolia (Lavandula angustifolia), Iran Iran (Cananga odorata), Geranium (Pelargonium asperum), manderin (Citrus reticulata), rosewood (Aniba rosaeodora), basil (Ocimum basilicum), fennel (Foeniculum vulgare), oregano (Origanum compactum), cypress (Cupressus sempervirens), orange sweet (Citrus sinensis), Lovin Tzara (Cinnamomum camphora), laurel (Laurus nobilis), eucalyptus radiata (Eucalyptus radiata).

### «Biological information generation method»

Next, the biological information generation method according to the present embodiment will be described. In the present embodiment (the biological information generation method, a biological information generation program to be described below, and a biological information generation apparatus to be described below), the types of the olfactory stimulants, the types of the fragrant molecules, the fragrant molecule classification method, and the action that the respective classified fragrant molecules give to the biological condition are exemplified in the principle related to the association between the smell and the biological condition. That is, in the present embodiment, the fragrant molecules are classified into the first to fourth classifications based on two characteristics (the height of the polarity of the fragrant molecule and the charged state of the corresponding fragrant molecule), but are not limited thereto. For example, the fragrant molecules may be classified by only the height of the polarity or only the charged state. Furthermore, the fragrant molecule classification method (also, association between the classification and the action on the living body) may be variously considered, and any method can be applied to the biological information generation method based on the present invention as long as the classification of the fragrant molecules and the action on the living body are associated with each other. For example, in the present example, the case of classification based on two axes is described as the classification of the fragrant molecules, but classification based on one axis or classification based on three or more axes may also be applied (in this way, the number of classifications of the fragrant molecules may be an arbitrary number of two or more by changing the number of axes of classification). Also, the number of classifications of the fragrant molecules and the types of the fragrant components (fragrant components as the main component corresponding to the olfactory stimulant) may not be matched with each other. For example, under the situation in which the fragrant molecules can be classified into the first classification, the second classification, the third classification, and the fourth classification, only two stimulants, that is, the olfactory stimulant having the fragrant component according to the first classification as the main component and olfactory stimulant having the fragrant component according to the second classification as the main component, may be used. The first classification and the second classification may be classified into the same classification, and the third classification and the fourth classification may be classified into the same classification (that is, classification focusing on only whether the charged state of the fragrant molecules is positive or negative), or the first classification and the fourth classification may be classified into the same classification, and the second classification and the third classification may be classified into the same classification (that is, classification focusing on only the polarity of the fragrant molecules)

The biological information generation method according to the present embodiment includes: (first step) classifying fragrant components capable of constituting an olfactory stimulant into first to fourth classifications, as a group including fragrant molecules, according to height of a polarity of a fragrant molecule included in the corresponding fragrant component and a charged state of the corresponding component; (second step) associating each of the first to fourth classifications with one or more actions that the fragrant component classified into each of the classifications gives to the biological condition; (third step) determining unpleasure degree given to a subject with respect to each of eight types of olfactory stimulants including, two for each, a first olfactory stimulant having the fragrant component classified into the first classification as the main component to a fourth olfactory stimulant having the fragrant component classified into the fourth classification as the main component; (fourth step) assigning a relatively high value to a correlation coefficient of the classification according to the main component in the corresponding type of the olfactory stimulant and one or more actions associated with the corresponding classification with respect to the type of the olfactory stimulant, whose unpleasure degree is determined as being relatively high in the third step; and (fifth step) generating graphic information for illustrating the biological condition of the subject with respect to each of the first to fourth classifications, based on the correlation coefficient. Hereinafter, each step and a biological information diagnosis method according to the present embodiment will be described in detail.

### <First step>

First, in the first step, the fragrant components capable of constituting the olfactory stimulant are classified into the first to fourth classifications according to the height of the polarity of the constituent fragrant molecule and the charged state of the corresponding fragrant molecule. Specifically, according to the above-described principle, the fragrant components are classified into the first classification, the second classification, the third classification, and the fourth classification, as the group including the fragrant molecules.

### <Second step>

Next, in the second step, each of the first to fourth classifications is associated with one or more actions that the fragrant component classified into each of the classifications gives to the biological condition. Specifically, as in the above-described principle, the fragrant component according to the first classification has a nerve balance recovery action; the fragrant component according to the second classification has a digestion promoting action; the fragrant component according to the third classification has a nerve tonic action; and the fragrant component according to the fourth classification has a stasis removal action.

### <Third step>

Next, in the third step, the unpleasure degree given to the subject is determined with respect to each of eight types of olfactory stimulants including, two for each, the first olfactory stimulant having the fragrant component classified into the first classification as the main component (as described above, the fragrant component according to the first classification is the group including the fragrant components classified into the first classification among the olfactory stimulants) to a fourth olfactory stimulant having the fragrant component classified into the fourth classification as the main component

Specifically, as a first process of the third step, eight types of olfactory stimulants are prepared, which include olfactory stimulants A and B having the fragrant component classified into the first classification as the main component of the smell, olfactory stimulants C and D having the fragrant component classified into the second classification as the main component of the smell, olfactory stimulants E and F having the fragrant component classified into the third classification as the main component of the smell, and olfactory stimulants G and H having the fragrant component classified into the fourth classification as the main component of the smell.

Next, as a second process of the third step, the subject takes a smell of any one of the olfactory stimulants A to H. Here, in the present embodiment, the subject first takes a smell of the olfactory stimulant A.

Next, as a third process of the third step, the subject takes a smell of the olfactory stimulant different from the first olfactory stimulant (olfactory stimulant A) among the olfactory stimulants A to H, and determines which one is an unpleasant smell. Here, it is assumed that when taking a smell of the olfactory stimulant B, the subject feels unpleasant to the olfactory stimulant A.

Next, as a fourth process of the third step, the action of the second process of the third step is repeated and ranking information of the olfactory stimulants A to H is stored in order in which the subject feels pleasant to the smell of the olfactory stimulants A to H (that is, not unpleasant). Here, it is assumed that the order in which the subject feels pleasant to the olfactory stimulants is H, G, F, E, D, C, B, and A (that is, the most unpleasant olfactory stimulant is A and the most pleasant olfactory stimulant is H).

In the present embodiment, the olfactory stimulants are eight types, and the olfactory stimulants each having two types of the fragrant components according to each classification (the first classification, the second classification, the third classification, and the fourth classification) as the main component are set. In such a case, stable data is derived as compared to a case where the olfactory stimulant has one type of each classified fragrant component as the main component (a total of four types of olfactory stimulants) (that is, in a case where a plurality of types of the fragrant components according to each classification is used, a temporary error hardly occurs). More stable data is derived by three types (or more types) of the fragrant components according to each classification. However, due to the increase in the number of trials, the burden of the subject in the diagnosis is increased and the processing of data becomes complicated.

Since the preference of the smell is subjective information and the degree thereof is different depending on individuals, it is difficult to measure and quantify information based on the preference of the smell. However, in the present embodiment, it is assumed that the information on the preference of the smell can be objectively measured to some degree by using relative information that is simply compared by individuals in order of preference of smell.

### <Fourth step>

Next, in the fourth step, a relatively high value is assigned to the correlation coefficient of the classification according to the main component in the corresponding type of the olfactory stimulant and the one or more actions associated with the corresponding classification with respect to the type of the olfactory stimulant, whose unpleasure degree is determined as being relatively high in the third step.

Specifically, first, based on the ranking information calculated in the third step, the ranking values (degree of unpleasure) of the olfactory stimulant A (degree of unpleasure: ranking No. 8, unpleasure degree: 8) and the olfactory stimulant B (degree of unpleasure: ranking No. 7, unpleasure degree: 7) are summed, and an unpleasure degree value according to the first classification (sum of the unpleasure frequencies) is calculated (here, the sum is 15). Similarly, the ranking values of the olfactory stimulants (olfactory stimulant C and olfactory stimulant D) having the fragrant component classified into the second classification as the main component are summed (the sum is 11), the ranking values of the olfactory stimulants (olfactory stimulant E and olfactory stimulant F) having the fragrant component classified into the third classification as the main component are summed (the sum is 7), and the ranking values of the olfactory stimulants (olfactory stimulant G and olfactory stimulant H) having the fragrant component classified into the fourth classification as the main component are summed (the sum is 3). In this manner, the unpleasure degree values according to the respective classifications are calculated.

In the fourth step of the present embodiment, in order to indicating the strength of the correlation between the classification according to the fragrant components being the main component in the olfactory stimulant and one or more actions associated with the corresponding classification, the sum of the unpleasure frequencies (unpleasure degree value) of the olfactory stimulant having the fragrant component according to the classification as the main component is calculated with respect to each classification. In this manner, in the present embodiment, as the unpleasure degree value is higher, it is determined that the correlation related to the action of the case where the fragrant molecule belonging to the corresponding classification is felt as a good smell is low (conversely, it is determined that the correlation related to the action of the case where the fragrant molecule belonging to the corresponding classification is felt as a bad smell is high). On the other hand, as the sum (unpleasure degree value) is lower, it is determined that the correlation related to the action of the case where the fragrant molecule belonging to the corresponding classification is felt as a good smell is high (conversely, it is determined that the correlation related to the action of the case where the fragrant molecule belonging to the corresponding classification is felt as a bad smell is low).

In the present embodiment, the rankings of the pleasure-unpleasure determination results of the respective olfactory stimulants are summed and the unpleasure degree value is calculated, with respect to each classification to which the fragrant components being the main component of the olfactory stimulant belong. However, the correlation coefficient of the classification according to the main component in the corresponding type of the olfactory stimulant and the one or more actions associated with the corresponding classification may be relatively changed according to the rankings of the pleasure-unpleasure determination results of the respective olfactory stimulants, and is not limited thereto (That is, when referencing the unpleasure degree according to a certain fragrant component, the action associated with the corresponding fragrant component may be relatively changed according to the unpleasure degree). For example, in the present example, the value related to the ranking information of the olfactory stimulant has a relatively high value with respect to the unpleasure (that is, the value of the ranking of the unpleasure degree is referenced as it is), but the unpleasure may have a relatively low value (that is, the reciprocal of the ranking of the unpleasure may be referenced).

### <Fifth step>

Next, in the fifth step, the graphic information for illustrating the biological condition of the subject is generated with respect to each of the first to fourth classifications, based on the correlation coefficient

Specifically, the graph (radar chart) is generated from the sum according to the first classification, the sum according to the second classification, the sum according to the third classification, and the sum according to the fourth classification.

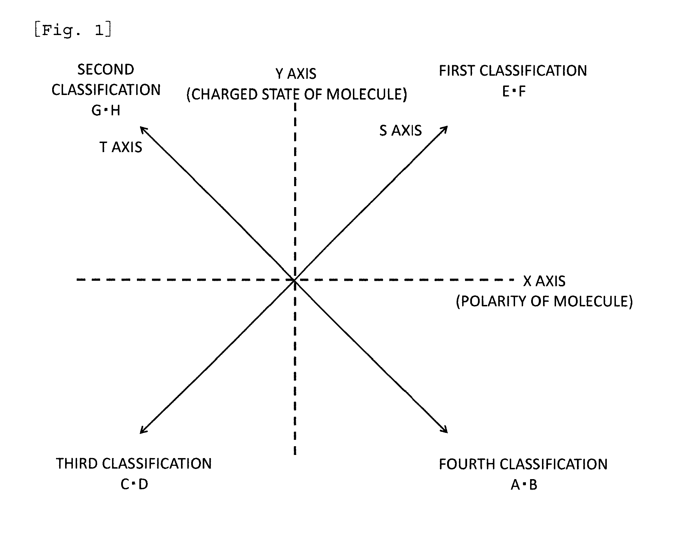

In the present example, the sum (unpleasure degree value) according to the first classification is 15, the sum according to the second classification is 11, the sum according to the third classification is 7, and the sum according to the fourth classification is 3. Therefore, as illustrated in Fig. 1, an ST orthogonal system is provided by rotating the XY orthogonal coordinate system in which the first to fourth classifications are assigned to the first to fourth quadrants, respectively (that is, the coordinate system as illustrated in Fig. 1) by 45 degrees around the X axis and the Y axis. In the ST orthogonal coordinate system, the point of (S, T) = (15, 0) as a first point according to the first classification is plotted. Similarly, a second point (0, 11) according to the second classification, a third point (-7, 0) according to the third classification, and a fourth point (0, -3) according to the fourth classification are plotted. Furthermore, the first to fourth points are connected to create a radar chart.

The graphic information for illustrating the biological condition in the fifth step is not limited to specific graphs, and indicates information capable of forming graphics (for example, one or more values or coordinate values associated with one or more parameters). Also, here, the rectangular radar chart is finally created, but this is only an example. As the final graphic information, various shapes can be considered according to purposes thereof. As an example, the shape is also changed according to the number of classifications of the fragrant molecules. For example, in a case where the number of classifications is three items, a triangular radar chart can be formed. In this way, polygonal graphic information can also be formed. When the polygonal graphic information (for example, radar chart) is formed, it is easy to visually understand the biological condition of the subject, but there is no problem even when the graphic information is formed in a point shape (scatter diagram shape), a line shape, a bar graph shape, a pie graph shape, a 3D graph shape, or the like.

Here, in the fifth step, as in the fourth step (step of assigning a relatively high value to the correlation coefficient of the classification according to the main component in the corresponding type of the olfactory stimulant and the one or more actions associated with the corresponding classification with respect to the type of the olfactory stimulant, whose unpleasure degree is determined as being relatively high in the third step), the correlation between the classification according to the main component in the olfactory stimulant and one or more actions associated with the corresponding classification may be generated as the graph information derived over time. In this case, since the information on the biological condition can be grasped over time, the long-term trend of the subject or the change in the short-term biological condition based on the long-term trend can be grasped.

### <Diagnosis method>

In the corresponding graph, the graph of the biological condition of the human is simply calculated from the unpleasure degree value according to the corresponding classification (here, the first classification, the second classification, the third classification, and the fourth classification) corresponding to the main component of the fragrant component included in the olfactory stimulant and the action associated with the corresponding classification, approximate trend of the information on the biological condition can be determined at a glance of the graph.

For example, a threshold value according to the unpleasure degree value (threshold value according to the action associated with each classification) is preset with respect to each classification, and the condition of the subject can be easily determined by determining whether the unpleasure degree value is higher or lower than the corresponding threshold value in each classification. For example, in the present embodiment, the threshold value of the unpleasure degree value is set as the average value (the average value is 9 in the present example), the balance of the biological condition is better as it is closer to the threshold value (that is, in a case where the unpleasure degree value according to a certain classification becomes the threshold value, the balance of the biological condition in the corresponding classification is the best), whether higher or lower than the corresponding average value of 9 is determined with respect to each classification (the first classification, the second classification, the third classification, and the fourth classification), in a case where the first classification (classification mainly related to the nerve balance recovery action) is higher than the first threshold value of 9, it can be regarded as a cold-hearted and calm condition and it is considered that there is a tendency to worry, and in a case where the first classification is lower than the first threshold value of 9, it is easy to break the balance of the nerve and it is considered as suffering from a disease of a nervous system and there is a restless tendency; in a case where the second classification (classification mainly related to the digestion promoting action) is higher than the second threshold value of 9, it is considered that there is a probability of overeating or excess of water important to promote digestion, it is easily swollen and easily cold, and a behavior is slow or an act is slow, and in a case where lower than the second threshold value of 9, it is considered that the digestive system is bad, the recovery of an injury or a cold is slow, and it is easy to suffer from stiff neck from the disorder of the stomach or the like; in a case where the third classification (classification mainly related to the nerve tonic action) is higher than the third threshold value of 9, it is considered that care is growing, there is a motivation, and the nerve is in an unstable condition, and in a case where lower than the third threshold value of 9, the mood is hardly uplifted and the motivation does not come out; and in a case where the fourth classification (classification mainly related to the stasis removal action) is higher than the fourth threshold value of 9, it is considered that metabolism is high and it is vigorous and active, and in a case where lower than the fourth threshold value of 9, a behavior is slow, a function of discharging excess water from the body is weak, and symptoms such as swelling, chronic fatigue, cold, fatigue tend to occur. In this manner, it is possible to grasp the biological condition of the subject. As the threshold values, a range may be set (for example, a threshold value may be set to 7 to 10), and in the case of corresponding to the corresponding range, the balance of the biological condition may be determined as good

In the interpretation of the unpleasure degree value, since the contradiction to the symptom and trend of the human occurs (for example, a condition of easily agitated and easily calmed can occur), there is no problem even when opposite properties are shown at the same time.

### <Purpose>

As described above, the essence of the present invention is that, when the information on the olfactory stimulant is set, the overview of the biological condition of the human can be easily grasped based on the pleasure-unpleasure condition corresponding to the olfactory stimulant and the information. That is, according to the present invention, the biological information on the biological condition can be easily known regardless of age or gender, the self biological condition can be freely diagnosed and the self constitution improvement (more broadly, self QOL improvement) can be performed with reference to the diagnosis result. By using the kit including a plurality of olfactory stimulants and the method according to the present invention, the self biological condition can be easily grasped regardless of place. Also, as the method of the constitution improvement (QOL improvement), regarding each classification, the threshold value set to each classification (9 in the above example) is determined as the best balanced condition. In order to approach the threshold value (for example, regarding the first classification, in a case where the unpleasure degree value is 12, the unpleasure degree value is reduced, and in a case where the unpleasure degree value is 3, the unpleasure degree value is increased), the improvement advice information (information appropriately including information on life style, such as diet or exercise preferable for the examinee, medicine, or aroma) may be presented (for example, the unpleasure degree value according to the first classification may be reduced so as to avoid taking ingredients containing a lot of fragrant components according to the first classification. Also, in particular, only the classification having a high unpleasure degree value (or, in particular, classification having a low unpleasure degree value) may be mainly referenced as the diagnosis information.

### «Biological information generation program»

Next, the biological information generation program according to the present embodiment will be described. The biological information generation program according to the present embodiment is a program for causing a computer to execute the biological information generation method according to the present embodiment, the biological information generation method including: a step (step I) of inputting determination results of unpleasure degree values with respect to four types of olfactory stimulants given to a subject; a step (step II) of assigning a relatively low or high value to a correlation coefficient of a classification according to a main component in the corresponding type of the olfactory stimulant and one or more actions associated with the corresponding classification with respect to the types of the olfactory stimulants having a relatively high unpleasure degree value in the input determination results; and a step (step III) of generating graphic information for illustrating a biological condition of the subject with respect to the first to fourth classifications, based on the correlation coefficient.

First, in the present program, the information (fragrant molecule information) on the respective fragrant molecules capable of constituting the olfactory stimulants classified into the first to fourth classifications according to the height of the polarity of the fragrant molecule and the charged state of the corresponding fragrant molecule, which is performed in the first step of the biological information generation method according to the present embodiment, and the information (action information) on the association between the first to fourth classifications and one or more actions that the fragrant molecules classified into the respective classifications give to the biological condition, which is performed in the second step of the biological information generation method according to the present embodiment, are prestored in a storage area (for example, a storage area of a processor, or a server connected through a network).

Prior to step I, the processor of the computer displays an unpleasure ranking information input form on an information display unit (for example, a monitor of a personal computer or a liquid crystal screen of a tablet).

### <Step I>

Next, in step I, the processor of the computer acquires the unpleasure ranking information (information on the ranking at which the subject feels good with respect to the smell of each olfactory stimulant), which is input in the unpleasure ranking information input form by an input unit (for example, a keyboard or a mouse, a touch panel, or the like), according to the present program, and temporarily stores the acquired unpleasure ranking information in a temporary storage area.

As such, step I is a step corresponding to the third step of the biological information generation method according to the present embodiment (details are the same as described above).

### <Step II>

Next, in step II, the processor of the computer derives the unpleasure degree value according to the fragrant component of each of the first to fourth classifications (the first classification, the second classification, the third classification, and the fourth classification), based on the unpleasure ranking information temporarily stored in the temporary storage area, according to the present program. Here, the unpleasure degree value is value information associated with the unpleasure ranking of each olfactory stimulant with respect to the classification, to which the corresponding fragrant component belongs, with reference to the fragrant component of each fragrant stimulant (in the present example, each olfactory has any one of fragrant components as a main component). In the present example, the unpleasure degree value becomes relatively larger as the unpleasure degree of the fragrant molecule according to the corresponding classification is increased, and becomes relatively smaller as the unpleasure degree of the fragrant molecule according to the corresponding classification is reduced {a low point is assigned to a more pleasant feeling (a higher point is assigned in order of unpleasure)}
As such, step II is a step corresponding to the fourth step of the biological information generation method according to the present embodiment (details are the same as described above).

### <Step III>

Next, in step III, the processor of the computer generates a graphic information image based on the unpleasure degree value derived in the second step according to the present program and displays the graphic information image on an information display unit.

As such, step III is a step corresponding to the fifth step of the biological information generation method according to the present embodiment (details are the same as described above).

### <Business model using biological information generation program>

By using the program according to the present invention, the method according to the present invention can be used on the Internet. Furthermore, a use method (business model) used for introducing products for improving the constitution of a subject with reference to graphic information (diagnosis result) of a biological condition of the subject may also be considered. Next, examples (first model and second model) of the business model using the biological information generation program according to the present invention are presented. Here, a model in which all programs according to the present invention are processed in a specific apparatus is presented as the first model. Next, a model in which a part of the program according to the present invention is processed in a specific apparatus and another program according to the present invention is processed on a web server is presented as the second model.

### <First Model>

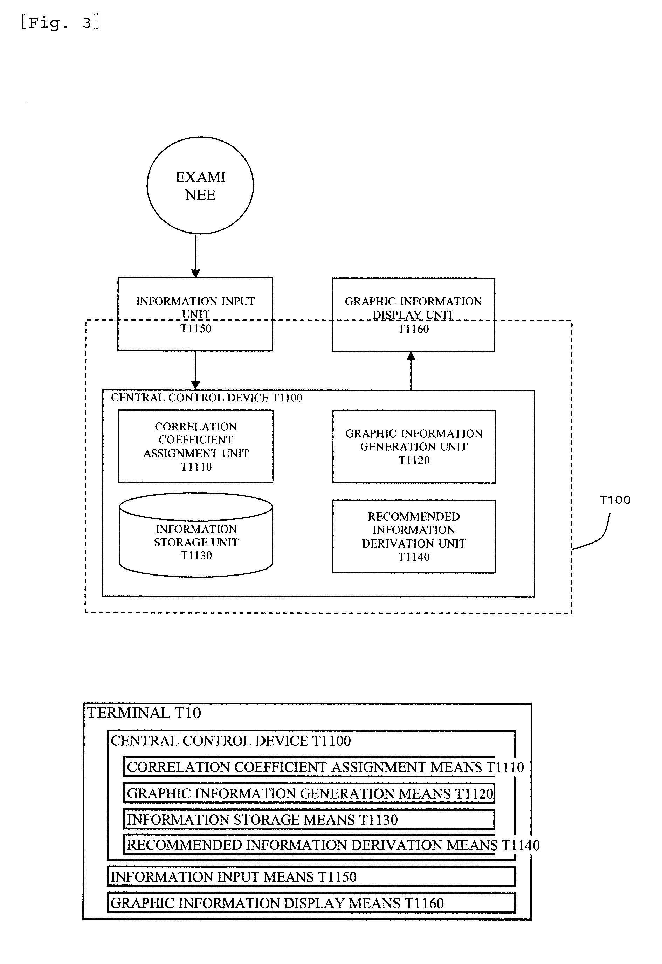

First, the function of a terminal T10 according to a first model will be described with reference to a block diagram of Fig. 3. First, the terminal T10 includes a central control device T1100 that functions as a program processing execution unit, an information input unit T1150 for inputting ranking information, and a graphic information display unit T1160 for displaying graphic information generated based on an unpleasure degree value. Also, the central control device T1100 further includes a correlation coefficient assignment unit T1110 that calculates the unpleasure degree value based on the ranking information, a graphic information generation unit T1120 that generates graphic information based on the unpleasure degree value, an information storage unit T1130 that previously stores information on types of olfactory stimulants or fragrant components being a main component constituting each olfactory stimulant, or information on the actions associated with the fragrant components as preliminary information, and a recommended product derivation unit T1140 that derives information on recommended products based on the unpleasure degree value corresponding to each classification.

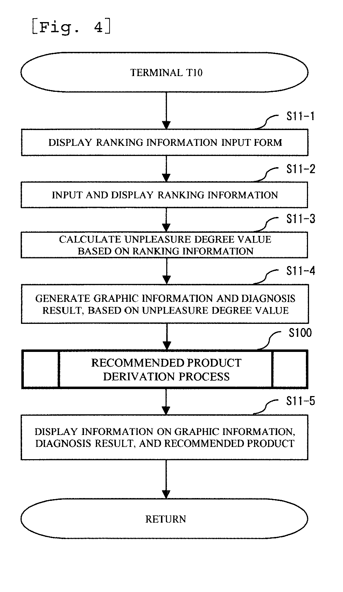

Next, Fig. 4 is a specific flowchart for providing information on recommended products that are available for constitution improvement or the like, based on diagnosis results, by using the biological information generation program according to the present invention.

First, in step 11-1, the central control device T1100 displays the ranking information input form on the display screen (for example, a display or a touch panel). Subsequently, in step 11-2, the ranking information is input to the central control device T1100 by the information input unit T1150 that can input information from the outside. Subsequently, in step 11-3, the correlation coefficient assignment unit T1110 calculates the unpleasure degree value based on the ranking information and the preliminary information previously stored in the information storage unit T1130. Subsequently, in step 11-4, the graphic information generation unit T1120 generates the graphic information and the diagnosis result based on the unpleasure degree value and the preliminary information previously stored in the information storage unit T1130. Subsequently, in step 100, the recommended product derivation unit T1140 executes a recommended product derivation process to be described below. Subsequently, in step 11-5, the graphic information display unit T1160, which can output the information to the outside of the central control device T1100, displays the graphic information, the diagnosis result, the information on the recommended product on the display screen.

In the present model, an apparatus with a display as the graphic information display unit is used to display the information input form and the final graphic information on the same display screen, but these may be displayed on separate display screens.

Also, the present mode is a model that presents information on the recommended product based on the diagnosis result, but is not limited thereto. Other recommended information {for example, the improvement device information (preferable life style such as diet or exercise, or a variety of information preferable for improving QOL of the examinee such as preferable medicine or preferable aroma)}.

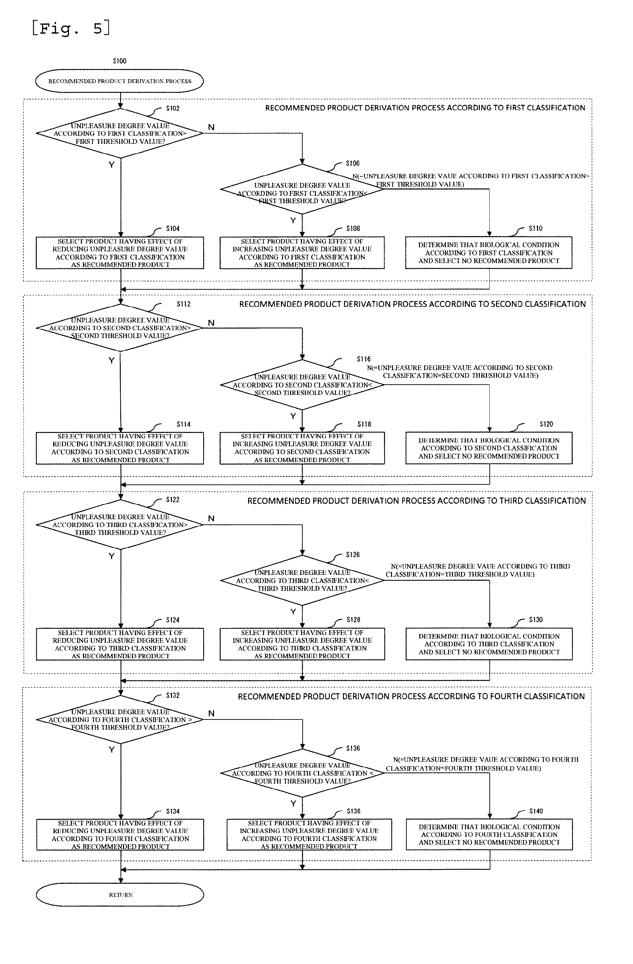

Next, Fig. 5 is a flowchart of a recommended product derivation process in step 100 of Fig. 4. First, in step 102, the recommended product derivation unit T1140 determines whether the unpleasure degree value according to the first classification is greater than the first threshold value. In step 104, when Yes in step 102, the recommended product derivation unit T1140 selects a product having an effect of reducing the unpleasure degree value according to the first classification as the recommended product and proceeds to step 112. On the other hand, in step 106, when No in step 102, the recommended product derivation unit T1140 determines whether the unpleasure degree value according to the first classification is smaller than the first threshold value. In step 108, when Yes in step 106, the recommended product derivation unit T1140 selects a product having an effect of increasing the unpleasure degree value according to the first classification as the recommended product and proceeds to step 112. On the other hand, in step 110, when No in step 106, that is, when the unpleasure degree value according to the first classification is the same as the threshold value, the recommended product derivation unit T1140 determines that the biological condition according to the first classification is normal, and proceeds to step 112, without selecting the recommended product. Note that, steps 102 to 110 are the product derivation process according to the first classification.

Subsequently, in step 112, the recommended product derivation unit T1140 determines whether the unpleasure degree value according to the second classification is greater than the second threshold value. In step 114, when Yes in step 112, the recommended product derivation unit T1140 selects a product having an effect of reducing the unpleasure degree value according to the second classification as the recommended product and proceeds to step 122. On the other hand, in step 116, when No in step 112, the recommended product derivation unit T1140 determines whether the unpleasure degree value according to the second classification is smaller than the second threshold value. In step 118, when Yes in step 116, the recommended product derivation unit T1140 selects a product having an effect of increasing the unpleasure degree value according to the second classification as the recommended product and proceeds to step 122. On the other hand, in step 120, when No in step 116, that is, when the unpleasure degree value according to the second classification is the same as the threshold value, the recommended product derivation unit T1140 determines that the biological condition according to the second classification is normal, and proceeds to step 122, without selecting the recommended product. Steps 112 to 120 are the product derivation process according to the second classification.

Subsequently, in step 122, the recommended product derivation unit T1140 determines whether the unpleasure degree value according to the third classification is greater than the third threshold value. In step 124, when Yes in step 122, the recommended product derivation unit T1140 selects a product having an effect of reducing the unpleasure degree value according to the third classification as the recommended product and proceeds to step 132. On the other hand, in step 126, when No in step 122, the recommended product derivation unit T1140 determines whether the unpleasure degree value according to the third classification is smaller than the third threshold value. In step 128, when Yes in step 126, the recommended product derivation unit T1140 selects a product having an effect of increasing the unpleasure degree value according to the third classification as the recommended product and proceeds to step 132. On the other hand, in step 130, when No in step 126, that is, when the unpleasure degree value according to the third classification is the same as the threshold value, the recommended product derivation unit T1140 determines that the biological condition according to the third classification is normal, and proceeds to step 132, without selecting the recommended product. Steps 122 to 130 are the product derivation process according to the third classification.

Subsequently, in step 132, the recommended product derivation unit T1140 determines whether the unpleasure degree value according to the fourth classification is greater than the fourth threshold value. In step 134, when Yes in step 132, the recommended product derivation unit T1140 selects a product having an effect of reducing the unpleasure degree value according to the fourth classification as the recommended product and proceeds subsequent processes (process of step 4-5). On the other hand, in step 136, when No in step 132, the recommended product derivation unit T1140 determines whether the unpleasure degree value according to the fourth classification is smaller than the fourth threshold value. In step 138, when Yes in step 136, the recommended product derivation unit T1140 selects a product having an effect of increasing the unpleasure degree value according to the fourth classification as the recommended product and proceeds subsequent processes (process of step 4-5). On the other hand, in step 140, when No in step 136, that is, when the unpleasure degree value according to the fourth classification is the same as the threshold value, the recommended product derivation unit T1140 determines that the biological condition according to the fourth classification is normal, and proceeds to subsequent process (process of step 4-5), without selecting the recommended product. Steps 132 to 140 are the product derivation process according to the fourth classification.

In the present model, the information storage unit T1130 may accumulate multiple times the graphic information, the diagnosis result, and the information on the recommended product, which are generated in steps 11-4 and 100, and the like. In this case, the terminal T10 according to the present model may further perform various processes (for example, a process of diagnosing the long-term biological condition trend and facilitating the redisplaying of the corresponding recommended product by bookmarking the previously derived recommended product), based on the accumulated information.

### <Second Mode>

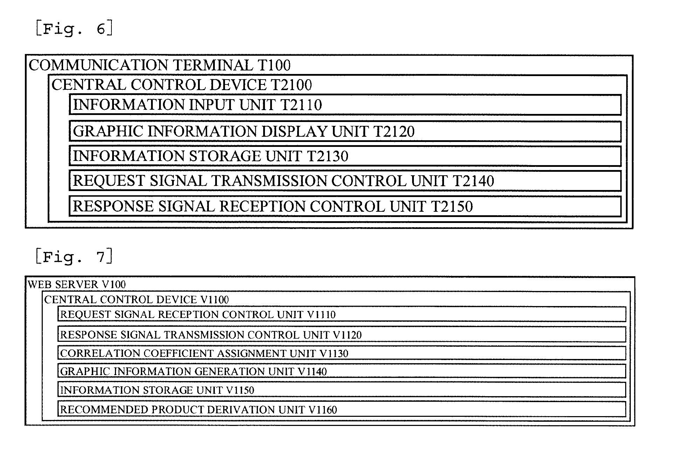

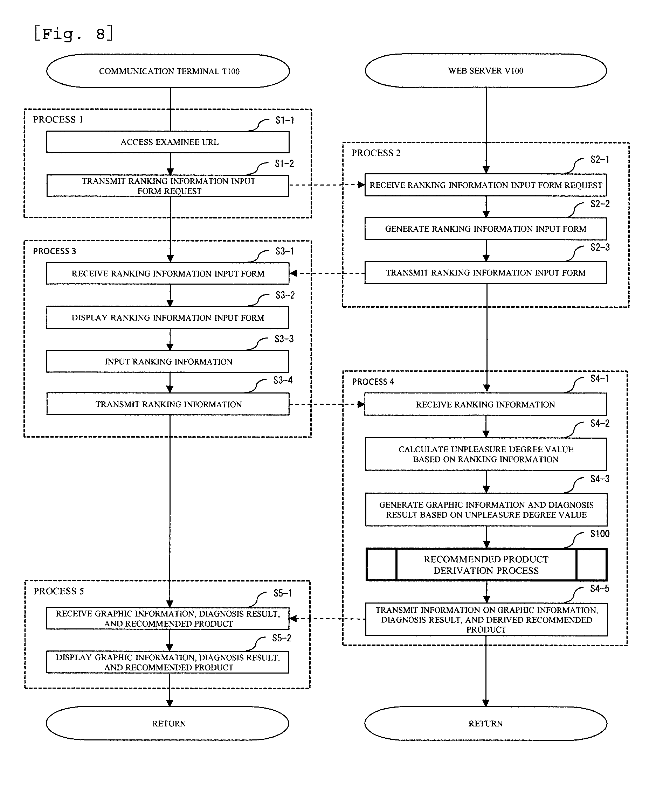

Next, a biological information generation program according to a second model will be described with reference to Figs. 6 to 8.

In the present model, information on the types of the olfactory stimulants or the fragrant components being the main component constituting the respective olfactory stimulants, or information on the actions associated with the classifications of the fragrant components is previously stored as preliminary information in the information storage unit V1150 of the web server.

First, Fig. 6 is a specific system flow for providing information on recommended products that are available for constitution improvement or the like, based on diagnosis results, by using the biological information generation program according to the present invention. First, a process of a communication terminal T100 is executed. First, in step 1-1, an examinee accesses an examinee URL by operating the communication terminal T100. Subsequently, in step 1-2, a request signal transmission control unit T2140 transmits a ranking information input form to a web server V100 side.

Subsequently, a process of the web server V100 is executed. First, in step 2-1, a request signal reception control unit V1110 receives a login information input form request transmitted from the communication terminal T100. Subsequently, in step 2-2, the central control device V1100 generates the ranking information input form (form for inputting each item related to the ranking information). Subsequently, in step 2-3, a response signal transmission control unit V1120 transmits the ranking information input form to the communication terminal T100.

Next, the process of the communication terminal T100 is executed. First, in step 3-1, a response signal reception control unit T2150 receives the ranking information input form request transmitted from the web server V100. Next, in step 3-2, the central control device T2100 displays a ranking information input form on a display screen. Subsequently, in step 3-3, the communication terminal T100 inputs the ranking information. Subsequently, in step 3-4, the request signal transmission control unit T2140 transmits the ranking information to the web server V100.

Subsequently, the process of the web server V100 is executed. First, in step 4-1, the request signal reception control unit V1110 receives the ranking information transmitted from the communication terminal T100. Subsequently, in step 4-2, the correlation coefficient assignment unit V1130 calculates the unpleasure degree value based on the ranking information and the preliminary information previously stored in the information storage unit V1150. Subsequently, in step 4-3, a graphic information generation unit V1140 generates the graphic information related to the biological condition and the diagnosis result related to the biological condition, based on the unpleasure degree value. Subsequently, in step 100, a recommended product derivation unit V1160 executes a recommended product derivation process. Since the recommended product derivation process is substantially the same as that of the first model, a description thereof will be omitted. Subsequently, in step 4-5, the response signal transmission control unit V1120 transmits the graphic information, the diagnosis result, and the derived recommended product to the communication terminal T100.

Next, the process of the communication terminal T100 is executed. First, in step 5-1, a response signal reception control unit T2150 receives the graphic information, the diagnosis result, and the recommended product, which are transmitted from the web server V100. Subsequently, in step 5-2, the graphic information display unit T2120 displays the graphic information, the diagnosis result, and the recommended product on the display screen.

### [Embodiment]

### «Execution Condition»

### <Material>

Essential oils A to H are prepared. As described below, each of the essential oils A to H is classified into one of four parameters (first classification, second classification, third classification, and fourth classification) by information on a polarity or a non-polarity and information on a positive state and a negative state of the fragrant component being the main component.

### (Essential oil E belonging to first classification)

Essential oil distilled from clary sage (Salvia sclarea flower cluster and foliage), a sample having linalyl acetate of esters as the main component of the fragrance

### (Essential oil F belonging to first classification)

Essential oil distilled from Chamomile Roman (Chamaemelum nobile flower), a sample having angelic acid isobutyl of esters as the main component of the fragrance

### (Essential oil G belonging to second classification)

Essential oil distilled from lemon grass (whole grass of Cymbopogon citratus), a sample having citral of terpene aldehydes (geranial, neral) as the main component of the fragrance

### (Essential oil H belonging to second classification)

Essential oil distilled from eucalyptus lemon (Eucalyptus citriodora leaves), a sample having citronellal of terpene aldehydes as the main component of the fragrance

### (Essential oil C belonging to third classification)

Essential oil distilled from rosewood (Aniba rosaeodora xylem), a sample having linalool of monoterpene alcohols as the main component of the fragrance

### (Essential oil D belonging to third classification)

Essential oil distilled from palmarosa (whole grass of Cymbopogon maritini), a sample having geraniol of monoterpene alcohols as the main component of the fragrance

### (Essential oil A belonging to fourth classification)

Essential oil distilled from Lovin Tzara (twig with Cinnamomum camphora leaves), a sample having 1,8 cineole of oxides as the main component of the fragrance

### (Essential oil B belonging to fourth classification)

Essential oil distilled from Akamatsu Europe (whole grass of Pinus sylvestris), a sample having α-pinene of monoterpene hydrocarbons as the main component of the fragrance
<Subject>
(Age)
30's
(Gender)
Female

### «Graph generation method»

### <Biological condition graph>

As a first step of a biological condition graph generation method at a specific time, determination results of pleasure-unpleasure that the essential oils A to H give to the subject and ranking information of olfactory stimulants based on the corresponding determination results are input as ranking information of the olfactory stimulants, and the corresponding ranking information is stored.

Specifically, first, as the first step, ranking information according to the pleasure-unpleasure degree related to the smells of the essential oils A to H is acquired. Specifically, first, the subject takes a smell of the essential oil A. Then, the subject takes a smell of the essential oil B and compares the unpleasure degrees of the essential oil A and the essential oil B. Similarly, the subject takes smells of all the essential oils A to H and determines the ranking of the unpleasure degrees of the essential oils A to H. In the present example, the order in which the smell is felt good is C, D, A, E, G, F, H, and B (Table 3). Then, as such, the information on the essential oil and the ranking of the pleasure-unpleasure (information on Table 3) is input to the input form of the personal computer.

**[Table 3]**

| RANKING OF PLEASANT FEELING | NO. 1 | NO. 2 | NO. 3 | NO. 4 | NO. 5 | NO. 6 | NO. 7 | NO. 8 |
|---|---|---|---|---|---|---|---|---|
| TYPE OF ESSENTIAL OIL | C | D | A | E | G | F | H | B |

Subsequently, as the second step, specific values (unpleasure degrees and unpleasure degree values) of the respective classifications for generating the biological condition graph in the personal computer are calculated. Specifically, in the present example, a high score is given (the unpleasure degree is high) as the ranking of the smell with good feeling is lower (that is, unpleasure). With respect to the essential oil C whose ranking (ranking of the smell with good feeling) is No. 1, one point is given to the unpleasure degree value according to the third classification to which the essential oil C belongs. With respect to the essential oil B whose ranking is No. 8, eight points are given to the unpleasure degree value according to the fourth classification to which the essential oil B belongs. In this manner, with respect to the unpleasure degree values according to the classifications (first classification, second classification, third classification, and fourth classification) to which the respective essential oils belong, the unpleasure degree values according to the respective classifications are calculated as illustrated in Table 4 below.

**[Table 4]**

| CLASSIFICATION | FIRST CLASSIFICATION | | SECOND CLASSIFICATION | | THIRD CLASSIFICATION | | FOURTH CLASSIFICATION | |
|---|---|---|---|---|---|---|---|---|
| TYPE OF ESSENTIAL OIL | E | F | G | H | C | D | A | B |
| RANKING | 4 | 6 | 5 | 7 | 1 | 2 | 3 | 8 |
| SCORE (UNPLEASURE DEGREE VALUE) | 10 | | 12 | | 3 | | 11 | |

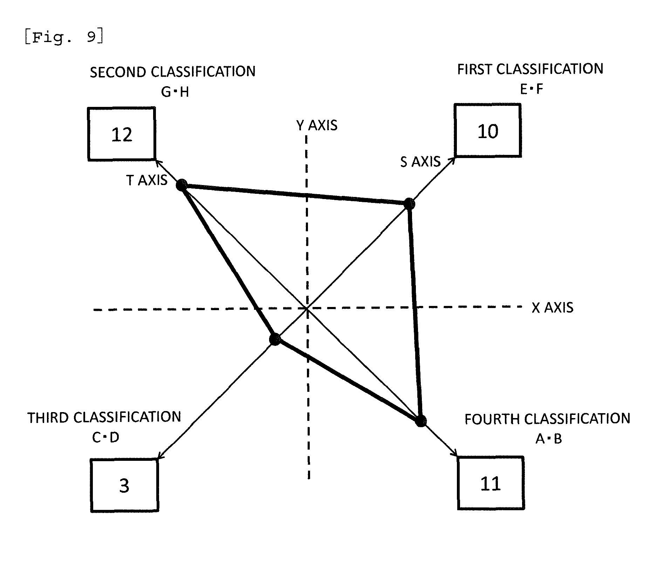

Subsequently, as the third step, the unpleasure degree values according to the respective classifications acquired in the second step are plotted on a graph. In the graph, in an XY orthogonal coordinate system in which an X-axis is information according to the polarity or non-polarity of the fragrant molecules and a Y axis is information according to the charged state of the fragrant molecules, an ST orthogonal coordinate system having axes rotated 45 degrees around the origin from the axes of the XY orthogonal coordinate system is set. Here, in the ST orthogonal coordinate system, the unpleasure degree values according to the first and third classifications are plotted on an S axis, and the unpleasure degree values according to the fourth and second classifications are plotted on a T axis. Specifically, on the axis of the ST orthogonal coordinate system that existed in the quadrants of the XY orthogonal coordinate system, points according to the respective parameters were plotted such that the distance from the origin became the unpleasure degree value according to the type, and the plotting on the graph was performed by connecting the corresponding points (Fig. 9).

### <Graph of change of biological condition over time>

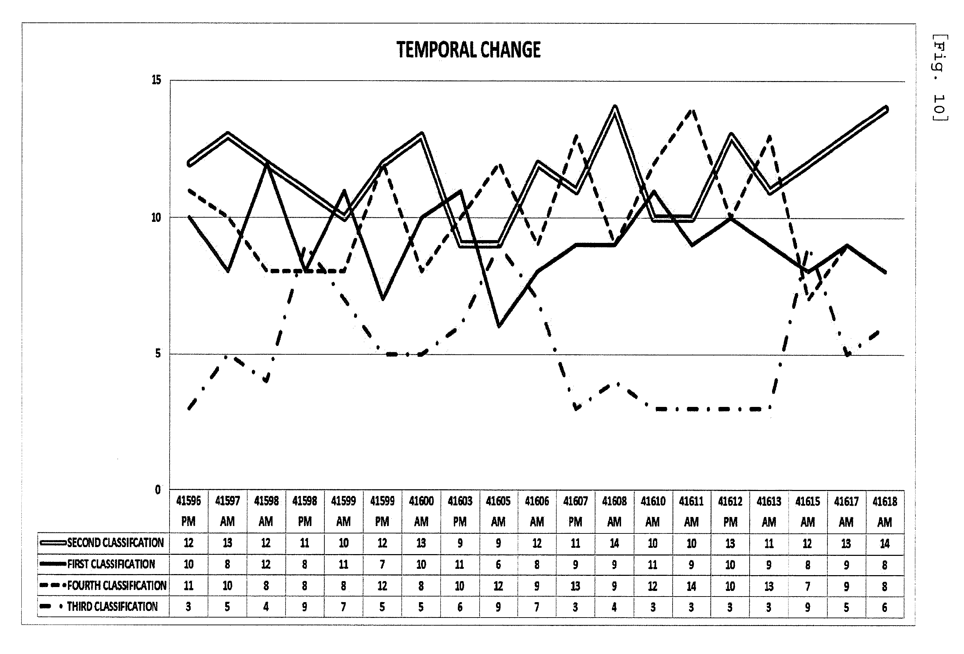

Next, as the graph of the change of the biological condition over time, over afternoon on November 18 to morning on December 10, the unpleasure degree values according to the respective classifications (first classification, second classification, third classification, and fourth classification) were calculated by the same method as the second step, and the change of the biological condition of the subject over time was plotted on the group by setting the horizontal axis as the number of days and setting the vertical axis as the unpleasure degree value (Fig. 10). In the drawing, a single line is the first classification, a double line is the second classification, a dashed-dotted line is the third classification, a dashed line is the fourth classification. Data on the first day in Fig. 10 corresponds to Fig. 9.

### <Evaluation>

Then, the biological condition of the subject was diagnosed using the biological condition graph generated by the graph generation method. In the present example, the calculated average value (average unpleasure degree value) of each classification (first classification, second classification, third classification, and fourth classification) is 9, and each parameter is somewhat apart from the calculated average value of 9. For example, the biological condition of the subject is determined as follows (that is, the calculated average value of 9 is set as the threshold value).

### (First classification)

In the case of less than 9: there is a tendency to be easy to forget or to be restless.

In the case of more than 9: there is a tendency to be easy to worry or depress.

### (Second classification)

In the case of less than 9: there is a tendency to be slow to recover a fatigue.

In the case of more than 9: there is a tendency to be easy to swell or susceptible to chronic fatigue.

### (Third classification)

In the case of less than 9: there is a tendency to be hard to uplift a mood.

In the case of more than 9: there is a tendency to be easy to uplift a mood.

### (Fourth classification)

In the case of less than 9: there is a tendency to be slow in action and to be low in metabolism.

In the case of more than 9: there is a tendency to active and vigorous but a body tends to be stiff.

From Fig. 9, the parameter according to the second classification is higher than the calculated average value, and the parameter according to the third classification is very lower than the calculated average value. Therefore, from Fig. 9, it can be seen that as the biological condition of the subject, it easily becomes fatigue, a body easily becomes stiff, a mood is not uplifted, and it becomes forgetful.

Here, when performing a hearing on the subject, the subject was troubled with stiff neck through the year, the subject was fatigue, and the subject could not sleep daily due to too much thinking. As such, it can be seen that a correlation between the tendency of the biological condition of the subject, which is read from the group, and the actual biological condition of the subject is high.

Also, when performing a hearing on the change of the biological condition over time (Fig. 10), it has proven that the subject was absent from a company on November 20, which is the third day after the start of the experiment. Also, on December 7, the company was a regular holiday. Furthermore, from November 25 to November 28, it has proven that the superior was on a business trip and thus was not in the company. Since these have no psychogenic stress, it is assumed that each classification becomes a value close to the threshold value, and the balance of the biological condition is well equipped to some extent (desired condition). In contrast, on November 30, the balance of the biological condition is collapsed, but a holiday work was done on the day. From the above, the day when the subject did not go to the company or did not face the superior, it could be read that the mind and body of the subject tended to be healthy. Also, the tendency to pay attention to the superior is matched with the fact that there is a bias on the axis of the days other than the day when the subject was absent from the company or the day when the subject did not face the superior.

## Claims

1. A biological information generation method, which generates graphic information for diagnosing a biological condition of a subject by using an olfactory stimulant, the biological information generation method comprising:
under a situation in which fragrant molecules are classified into first to M^{th} classifications (where M is a predetermined integer equal to or greater than 2) according to a height of a polarity and a charged state,
a first step of classifying the respective fragrant components capable of constituting olfactory stimulants into first to N classifications (where N is a predetermined integer equal to or greater than 2 and equal to or less than M) as a group including the fragrant molecules;
a second step of associating each of the first to N^{th} classifications with one or more actions that a fragrant component classified into each of the classifications gives to a biological condition;
a third step of determining an unpleasure degree for olfactory stimulation given to the subject for each of the N types of olfactory stimulants, with respect to at least N types of the olfactory stimulants including a first olfactory stimulant having the fragrant component classified into the first classification as a main component to an N^{th} olfactory stimulant having the fragrant component classified into the N^{th} classification as a main component;
regarding the olfactory stimulant that unpleasure degree in the determining is a relatively high type, a fourth step of assigning a relatively high value or a relatively low value to a correlation coefficient of between the classification, to which the main component in the olfactory stimulant of the type belongs, and the one or more actions associated with the classification; and
a fifth step of generating graphic information for illustrating the biological condition of the subject with respect to each of the first to N^{th} classifications, based on the correlation coefficient.

2. A program for causing a computer to execute the biological information generation method according to claim 1, the program comprising:
a step of inputting an unpleasure degree determination result of olfactory stimulation given to the subject with respect to each of the N types of the olfactory stimulants;
regarding the olfactory stimulant that unpleasure degree input in the determination result is a relatively high type, a step of assigning a relatively high value or a relatively low value to the correlation coefficient of between the classification, to which the main component in the olfactory stimulant of the type belongs, and the one or more actions associated with the classification; and
a step of generating the graphic information for illustrating the biological condition of the subject with respect to each of the first to N^{th} classifications, based on the correlation coefficient.

3. An apparatus for executing the biological information generation method according to claim 1, the apparatus comprising:
an information input unit capable of inputting an unpleasure degree determination result of olfactory stimulation given to the subject with respect to each of the N types of the olfactory stimulants;
a correlation coefficient assignment unit, regarding the olfactory stimulant that unpleasure degree in the input determination result is a relatively high type, for assigning a relatively high value or a relatively low value to the correlation coefficient of between the classification, to which the main component in the olfactory stimulant of the type belongs, and the one or more actions associated with the classification; and
a graphic information generation unit for generating the graphic information for illustrating the biological condition of the subject with respect to each of the first to N^{th} classifications, based on the correlation coefficient.
